# EUROPEAN PATENT SPECIFICATION

(11) **EP 0 672 509 B1**
(45) Date of publication and mention of the grant of the patent: **01.09.1999**
(21) Application number: 95301688.8
(22) Date of filing: 14.03.1995
(51) Int. Cl.: B29C 41/22, A61B 19/04, B65D 55/06, B29C 41/14

(54) **Rubber articles**
Gummiartikel
Objets en caoutchouc

(30) Priority: 15.03.1994 GB 9404975
(43) Date of publication of application: 20.09.1995
(73) Proprietor: Richardson, Margaret Pamela, Saundersfoot SA69 9EA (GB); Richardson, Philip, Saundersfoot SA69 9EA (GB)
(72) Inventor: Richardson, Margaret Pamela, Saundersfoot SA69 9EA (GB); Richardson, Philip, Saundersfoot SA69 9EA (GB)
(74) Representative: Austin, Hedley William

(56) References cited:
- EP-A- 0 368 456
- EP-A- 0 557 625
- EP-A- 0 629 497
- WO-A-94/02080
- GB-A- 2 243 825
- US-A- 5 017 427

## Description

The present invention is concerned with the production of multi-layer rubber articles.

Multi-layer rubber articles are known. A problem often experienced during production and use of such multi-layer articles is that the individual layers adhere to adjacent layers which can be detrimental to certain properties of the articles, and it is generally difficult to detect breach of the articles during manufacture thereof. We have previously described in our GB Patent Specification 2242817 a two-layer rubber article having an outer layer of translucent material and an inner layer of darker colour, such that on breach of the article, there may be a change of perceived colour as a result of entry of liquid between the layers.

We have now developed a method of preparation of such articles which helps to alleviate the problems associated with the production and use of known multi-layer rubber articles.

According to the present invention there is provided a method of producing a rubber article, which method comprises:
a) applying a liquid containing an elastomer to a former having a release surface so as to form a first body;
b) applying a separation material to at least part of the surface of the elastomer present on said former so as to form a separable coating thereon;
c) applying a liquid containing an elastomer onto the separation material present on said first body on said former to form a further body at least partly covering said first body;
d) at least partly curing said elastomers; and
e) removing said bodies jointly from said former, so as to provide an article having an outer layer of translucent material and an adjacent inner coloured layer of darker colour than the outer layer, with said separation material therebetween, said separation material being such as to permit capillary action between said outer layer and said inner layer on breach of one or both of said layers.

According to a first embodiment of the present invention, the rubber article comprises a glove. According to a second embodiment, the rubber article comprises a condom, or a closed end portion of a condom. It may be preferred that the whole condom comprises inner and outer layers as hereinbefore described; alternatively it may be preferred that it is only the closed end portion of the condom which is two-layered. It is appreciated that the present invention has further applications in respect of other multi-layer rubber articles, such as packaging or the like.

Judgement by eye of the difference between lighter and darker shades of colour is generally straightforward. However, if needed, guidance can be gained from the international system of colour definition known as the "NCS". The NCS is described in the "ICI Colour Dimensions Colour Atlas" published by Imperial Chemical Industries Plc of London in 1986 ("Colour Dimensions" is a registered trade mark of Imperial Chemical Industries). The NCS defines colour in terms of a cypher, the first two digits of which extend from 00 to 99 with 00 representing white (that is, the ultimate lightness) and 99 representing black with the intermediate values from 01 to 98 representing increasingly darker shades. Lighter shades therefore have a lower pair of first digits. It is preferred that the outer layer has an NCS value at least 10 units lower than that of the inner layer.

It may be preferred that the separation material comprises a gelatinous medium such as a hydrogel or a resinous emulsion. The gelatinous medium may contain a coagulant such as calcium chloride or calcium nitrate or the like, and/or particulate solid material such as calcium carbonate.

Such a coagulant may contain a dispersion of one or more ingredients present in the elastomeric latex of one or more of the bodies. Typical such ingredients include micronised sulfur, zinc oxide or other similar finely divided solid material, and may advantageously further comprise a film-forming polymeric stabiliser or binder such as polyvinyl acetate or the like (in order to inhibit generation of particulates on damage to the rubber article). These ingredients, when present in the coagulant, may advantageously comprise one or more of the facing surfaces of the bodies, in such a way as to effect separation of the bodies and allow liquid penetration therebetween. The coagulant may contain a non-film forming particulate material, such as diatomaceous earth, bentonite or the like, suitable for enhancing the required separation of the bodies.

Preferably the separation material may comprise a water-absorbent particulate mineral medium, such as calcium carbonate, Celite (a material commercially available under the trade mark Lytron 820 from Manville-Schurer Corp), diatomaceous earth or the like. The use of the above-described particulate materials is advantageous because of their adhesion to the latices of the bodies, their water absorbent properties and interlocking structure being believed to obviate release thereof from a resulting article in the case of rupture of one or both of the bodies.

The method according to the invention may advantageously involve application of one or more latex modifying agents to at least part of the surface of one or both of the bodies. The agent may be present in a coagulant of the separation material; alternatively it may be applied directly to a body.

A preferred latex modifying agent comprises cetyl pyridinium chloride, the use of which is also advantageous because of the anti-viral properties thereof. It is sometimes preferred that the cetyl pyridinium chloride is applied in an alcoholic solution to at least part of the surface of a body.

The latex modifying agent may alternatively comprise an exudate which has migrated from the bulk of an elastomeric latex, of one or both of the bodies, to the surface thereof by the process of syneresis. At least one of the elastomeric latices may be selected so as to include a suitable exudate. Alternatively, the method may include impregnating at least one of the latices with a suitable exudate, such as an emulsion of a low molecular weight wax, a pH dependent chemical solution or the like. Preferably the method includes drying of the bodies, so as to permit migration of the exudate to the surface of at least one of the bodies.

Advantageously, the method involves a prevulcanisation stage, which is beneficial in optimising the desired properties of the resultant article, such as wet gel strength, on-mould shrinkage, moisture retention during vulcanisation, physical strength and the like. The prevulcanisation step preferably involves monitoring of the swelling index of the latices in order to enable optimisation of the above properties.

In a preferred embodiment of the invention, the method may involve respective prevulcanisation of the first and second bodies to varying extents. Typically the latex material of the inner body (in use) may have a swelling index of about 1.8, whereas that of the outer body may have a swelling index of about 2.1; however variance of these values may be made to achieve optimum separation between the layers.

The lower the swelling index, the greater the level of the cross-linkage of the latex; a high level of cross linkage gives rise to an increased level of syneresis as described above.

It is preferred that the separation material is applied as a substantially discontinuous layer to the first body. The nature of the separation material is preferably such that the elastomeric bodies preferentially adhere to the separation material rather than to each other; such preferential adherence promotes air gap formation between the layers and enhances detection of any liquid penetration between the layers. It is further preferred that the separation material can act as a release agent which eases separation of the inner and outer layers.

The separation material may in some embodiments include an anti-bacterial or anti-viral material, such as the material commercially available under the trade mark Nonoxynol 9.

It is preferred that the resulting article is substantially free of any colorimetric indicator material which would be responsive to the penetration of body or any other fluids between the layers. Any visual detection of damage or breach of the article by body or other fluids is due to capillary action between the two layers which causes the inner layer to become substantially contiguous with the outer layer in the area of breach such that the perceived colour is that of the inner layer.

The liquid containing an elastomer is generally applied to the former by dipping, although other methods aimed at substantially complete coverage of the former (such as spraying or the like) may be used in some instances. The liquid is typically a rubber latex, which may be applied to the former after application of a coagulant to a former.

After application to the former, the elastomeric membrane thus produced is generally dipped in one or more baths, such as further coagulant, water wash, vulcanisation bath and optional further coating, such as a lubricant or hydrogel.

The outer layer of the finished article is of a translucent material and has a contrasting colour relative to the inner layer. Preferably the inner layer should be of substantially uniform coloration throughout. Typically, the outer layer may be of yellow or white translucent material and the inner layer of a darker colour, such as green, black or the like.

The former is typically of porcelain or the like, as is conventionally used for dip-forming rubber articles. It may be preferred that at least a portion of the surface of the former is of a dark colour which allows the integrity of a translucent elastomeric layer present on the former to be tested in situ.

If a perforation, puncture or small rupture exists communicating between the outer and inner surfaces of the translucent layer, the liquid contacting the layer's outer surface may pass through the perforation and contact with the outer surface of such a porcelain former, thereby giving a change in the perceived colour from pale (the translucent layer) to dark (the coloured former).

The inner and outer layers of the rubber article may be sealed together in the method according to the invention; when such sealing is employed it is preferably at or near an open end region of the article, the sealing preferably being such that a substantial portion of the outer layer is substantially unbonded to the inner layer.

The invention may be more clearly understood from the following description and accompanying drawings, given by way of example only, wherein;
Figure 1 is a sectional view of an exemplary glove according to the invention; and
Figure 2 shows, diagrammatically, a method according to the invention.

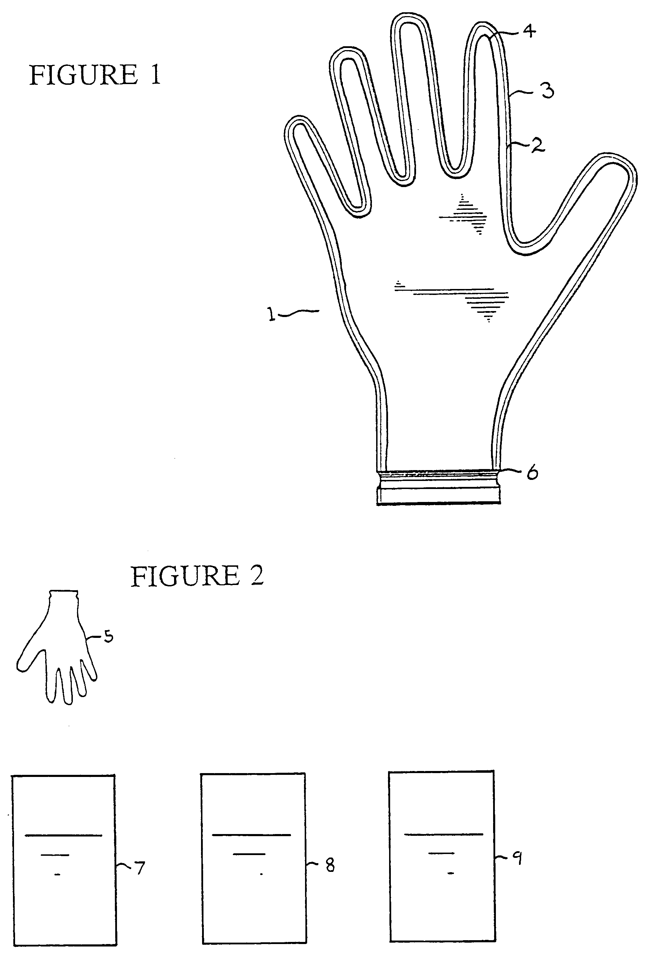

Referring to Figure 1, there is illustrated a glove indicated generally by the reference numeral 1, which comprises an inner glove 4 of latex, which may be highly coloured, for example, of black, luminous yellow, or green. The outer surface of inner glove 4 may be smooth or may have a fine textured finish. An outer (translucent) glove 3 of contrasting colour relative to the inner glove is of the same shape and size as the inner glove and is a close fit thereon and sealed thereto at the wrist portion 6. Between the inner glove 4 and outer glove 3 is a separation layer 2 which keeps the two gloves separated from each other. At or near the edges of the gloves 4,3 which engage the wearer's wrist, the gloves 4,3 may be sealed together. The space between the gloves 4,3 is substantially evacuated of air, so that the adjacent surfaces of the gloves 4,3 are pressed firmly against the separation material which is situated between the two gloves, the inner and outer gloves 4,3 act as a single glove.

However, if a small puncture or leak is made in either of the gloves 4,3 in the presence of aqueous liquids such as blood or body fluids, a colour change in the vicinity of the puncture becomes apparent, indicating the existence of the puncture or leak. At the same time, the outer glove 3 becomes relatively more mobile over the inner glove 4 in the vicinity of the puncture, causing a detectable change of feel of the glove.

An anti-bacterial or anti-viral substance may be present in the space between the gloves 4,3 to act against any viral material which penetrates into the space.

For some applications, more than two gloves 4,3 may be provided, one inside the other, all sealed together at the wrist-engaging edges and having the spaces between the gloves air-free.

Referring to Figure 2, there is provided a diagrammatic illustration of the production of the latex glove as described above. A porcelain former 5 is dipped into a first container 7 of latex so as to form the first layer of the glove 1. The former 5 is then dipped into a second container having therein a separation material, such as calcium carbonate or diatomaceous earth. The former 5 is then dipped into a further container 9 of latex, thereby forming a second latex layer on the outside of the separating material 2. The latex is then cured, and further subjected to a water wash, vulcanisation and optionally treatment with a lubricant (not shown). The inner layer on the former is translucent, in contrast to the darker outer layer. The inner and outer layers respectively, are then sealed at the end nearest the wrist portion of the glove 1. Because the glove 1 as a result of the method is formed inside out on the former 5, it must then be everted. Therefore the outer layer becomes inner glove 4 and the inner layer becomes outer glove 3.

## Claims

1. A method of producing a rubber article, which method comprises:
a) applying a liquid containing an elastomer to a former so as to form a first body, said former having a release surface;
b) applying a separation material to at least part of the surface of said elastomer present on said former so as to form a separable coating thereon;
c) applying a liquid containing an elastomer onto the separation material present on said first body on said former to form a further body at least partly covering said first body;
d) at least partly curing said elastomers; and
e) removing said bodies jointly from said former, so as to provide an article having an outer layer of translucent material and an adjacent inner coloured layer of darker colour than said outer layer, with said separation material therebetween, said separation material being such as to permit capillary action between said outer layer and said inner layer on breach of one or both of said layers.

2. A method according to claim 1, wherein said separation material comprises a gelatinous medium and optionally a coagulant, which coagulant may include a particulate solid material.

3. A method according to claim 1 or 2, further comprising application of one or more latex modifying agents, such as cetyl pyridinium chloride, in an alcoholic solution, to at least part of the surface of one or both of said bodies.

4. A method according to claim 1, wherein said separation material contains an anti-bacterial or anti-viral material.

5. A method according to any preceding claim, wherein said rubber article is substantially free of any colorimetric indicator material which would be responsive to the penetration of body or any other fluids between said layers.

6. A method according to any preceding claim, wherein said outer layer is of a translucent material and has a contrasting colour relative to said inner layer, which inner layer preferably has a substantially uniform coloration throughout.

7. A method according to any preceding claim, wherein said former is of porcelain, wherein at least a portion of the surface of said former is of a darker colour relative to the natural colour of porcelain.

8. A method according to claim 6 or 7, wherein said inner and outer layers of said rubber article are sealed together, typically adjacent an open end region of said article, and/or such that a substantial portion of said outer layer is substantially unbonded to said inner layer.

9. A method according to any preceding claim, wherein said rubber article comprises a glove, a condom, or a closed end portion of a condom.

## Patentansprüche

1. Verfahren zur Herstellung eines Gummiartikels, wobei das Verfahren umfaßt:
a) Das Aufbringen einer Flüssigkeit, die ein Elastomer enthält, auf einen Formgeber, so daß ein erster Körper geformt wird, wobei der Formgeber eine Ablöse-Oberfläche besitzt;
b) das Aufbringen eines Trennmaterials auf wenigstens einen Teil der Oberfläche des auf dem Formgeber befindlichen Elastomers, so daß eine abtrennbare Beschichtung darauf geformt wird;
c) das Aufbringen einer Flüssigkeit, die ein Elastomer enthält, auf das Trennmaterial, das auf dem sich auf dem Formgeber befindenden ersten Körper vorliegt, um einen weiteren Körper zu formen, der zumindest teilweise den ersten Körper bedeckt;
d) das mindestens teilweise Aushärten der Elastomere; und
e) das Entfernen der Körper zusammenhängend vom Formgeber, so daß ein Artikel bereitgestellt wird mit einer Außenschicht aus durchscheinendem Material und einer benachbarten gefärbten Innenschicht von dunklerer Färbung als die Außenschicht, wobei das Trennmaterial dazwischenliegt und so beschaffen ist, daß zwischen der Außenschicht und der Innenschicht bei Bruch (Riß) einer oder beider Schichten Kapillarkräfte wirken können.

2. Verfahren nach Anspruch 1, in dem das Trennmaterial ein gelatinöses Medium und optional ein Koagulans umfaßt, wobei das Koagulans einen partikulösen Feststoff enthalten kann.

3. Verfahren nach Anspruch 1 oder 2, weiterhin umfassend die Applikation von einem oder mehreren Latex-modifizierenden Agenzien, wie beispielsweise Cetylpyridiniumchlorid, in alkoholischer Lösung, auf wenigstens einen Teil der Oberfläche eines oder beider Körper.

4. Verfahren nach Anspruch 1, wobei das Trennmaterial ein antibakterielles oder antivirales Material enthält.

5. Verfahren nach einem der vorangehenden Ansprüche, wobei der Gummiartikel im wesentlichen frei ist von colorimetrischem Indikatormaterial, das auf die Penetration von Körper- oder anderen Flüssigkeiten (Fluiden) zwischen den Schichten reagieren würde.

6. Verfahren nach einem der vorangehenden Ansprüche, wobei die Außenschicht aus durchscheinendem Material besteht und eine relativ zur Innenschicht kontrastierende Farbe aufweist, und die Innenschicht vorzugsweise durchgehend eine im wesentlichen einheitliche Färbung aufweist.

7. Verfahren nach einem der vorangehenden Ansprüche, wobei der Formgeber aus Porzellan besteht, wobei zumindest ein Teil der Oberfläche des Formgebers eine dunklere Farbe als die natürliche Farbe von Porzellan aufweist.

8. Verfahren nach Anspruch 6 oder 7, wobei die Innenschicht und die Außenschicht des Gummiartikels aneinandergesiegelt sind, typischerweise neben einer Öffnungs-Endregion des Artikels und/oder so, daß ein wesentlicher Bereich der Außenschicht im wesentlichen nicht an die Innenschicht gebunden ist.

9. Verfahren nach einem der vorangehenden Ansprüche, wobei der Gummiartikel einen Handschuh, ein Kondom oder einen geschlossenen Endabschnitt eines Kondoms umfaßt.

## Revendications

1. Procédé pour produire un objet en caoutchouc, lequel procédé comprend :
a) l'application d'un liquide contenant un élastomère sur un gabarit de façon à former un premier corps, ledit gabarit ayant une surface lubrifiée ;
b) l'application d'un matériau de séparation sur au moins une partie de la surface dudit élastomère présent sur ledit gabarit de façon à former une couche séparable dessus ;
c) l'application d'un liquide contenant un élastomère sur le matériau de séparation présent sur ledit premier corps sur ledit gabarit pour former un corps supplémentaire qui couvre au moins partiellement ledit premier corps ;
d) la réticulation au moins partielle desdits élastomères ; et
e) le retrait de façon jointe desdits corps dudit gabarit, de façon à fournir un objet ayant une couche externe de matériau translucide et une couche interne adjacente colorée de couleur plus sombre que ladite couche externe, avec ledit matériau de séparation entre les deux, ledit matériau de séparation étant tel qu'il permet une action de capillarité entre ladite couche externe et ladite couche interne dans le cas où l'une des deux couches ou les deux couches se fendent.

2. Procédé selon la revendication 1, caractérisé en ce que ledit matériau de séparation est composé d'un milieu gélatineux et facultativement d'un coagulant, lequel coagulant peut contenir un matériau solide particulaire.

3. Procédé selon l'une quelconque des revendications 1 ou 2, comportant de plus l'application d'un ou plusieurs agents modifiant le latex, comme le chlorure de cétyl pyridinium, en solution alcoolique à au moins une partie de la surface de l'un des deux ou des deux dits corps.

4. Procédé selon la revendication 1, caractérisé en ce que ledit matériau de séparation contient une matière anti-bactériale ou anti-virale.

5. Procédé selon l'une quelconque des revendications précédentes, caractérisé en ce que ledit objet en caoutchouc est substantiellement dépourvu de tout indicateur colorimétrique qui réagirait à la pénétration de tout fluide corporel ou de tout autre fluide entre lesdites couches.

6. Procédé selon l'une quelconque des revendications précédentes, caractérisé en ce que ladite couche externe est composée d'un matériau translucide et a une couleur qui contraste avec celle de la couche interne, laquelle couche interne a de préférence une couleur substantiellement uniforme sur toute sa surface.

7. Procédé selon l'une quelconque des revendications précédentes, caractérisé en ce que ledit gabarit est en porcelaine, caractérisé en ce que au moins une partie de la surface dudit gabarit est d'une couleur plus sombre que la couleur naturelle de la porcelaine.

8. Procédé selon l'une quelconque des revendications 6 ou 7, caractérisé en ce que les couches interne et externe dudit objet ci caoutchouc sont scellées hermétiquement l'une à l'autre, caractéristiquement de façon adjacente à une extrémité ouverte dudit objet, et/ou de façon à ce qu'une part importante de ladite couche externe soit substantiellement non liée à ladite couche interne.

9. Procédé selon l'une quelconque des revendications précédentes, caractérisé en ce que ledit objet en caoutchouc est un gant, un préservatif, ou l'extrémité fermée d'un préservatif.
